# EUROPÄISCHE PATENTSCHRIFT

(11) **EP 3 447 119 B1**
(45) Veröffentlichungstag und Bekanntmachung des Hinweises auf die Patenterteilung: **17.06.2020**
(21) Anmeldenummer: 18187474.4
(22) Anmeldetag: 06.08.2018
(51) Int. Cl.: C12M 1/107, C12M 1/00, C12M 1/34

(54) **BIOGASANLAGE SOWIE EINRICHTUNG UND VERFAHREN ZUM EINTRAGEN VON ZUSATZSTOFFEN IN EINEN FERMENTER**
BIOGAS INSTALLATION AND DEVICE AND METHOD FOR INSERTING ADDITIVES INTO A FERMENTER
INSTALLATION DE BIOGAZ AINSI QUE DISPOSITIF ET PROCÉDÉ D'INTRODUCTION D'ADDITIFS DANS UN FERMENTATEUR

(30) Priorität: 25.08.2017 DE 102017119608
(43) Veröffentlichungstag der Anmeldung: 27.02.2019
(73) Patentinhaber: E.ON Bioerdgas GmbH, 45131 Essen (DE)
(72) Erfinder: Deupmann, Hermann, 48432 Rheine (DE); Freiherr von Canstein, Harald, 40667 Meerbusch (DE)
(74) Vertreter: Harlacher, Mechthild

(56) Entgegenhaltungen:
- EP-A2- 1 997 901
- WO-A1-2009/077623
- US-A1- 2007 184 542

## Beschreibung

Die Erfindung betrifft eine Biogasanlage, mit mindestens einem Fermenter für den Abbau eines Substrats zum Erzeugen von Biogas, wobei an den Fermenter mindestens eine Rohrleitung zum Zuführen eines pumpfähigen Mediums in den Fermenter angeschlossen ist.

Ferner betrifft die Erfindung eine Einrichtung und ein Verfahren zum Eintragen von Zusatzstoffen in einen Fermenter einer Biogasanlage, wobei an den Fermenter mindestens eine Rohrleitung zum Zuführen eines pumpfähigen Mediums in den Fermenter angeschlossen ist.

Das Erzeugen von Biogas aus einem Substrat ist eine der wesentlichen Technologien der regenerativen Energieerzeugung. Unter Substrat wird Biomasse aus biologisch abbaubaren Stoffen verstanden. Bei dem Substrat kann es sich um nachwachsende Rohstoffe oder um Abfälle wie Tiermist oder Gülle handeln.

Es gibt verschiedene Anlagentypen, die alle mindestens einen Fermenter aufweisen, in dem das zugeführte Substrat vergoren wird. Abhängig von den Fermentertypen und der Anlagengröße hat eine Anlage einen oder mehrere Fermenter, in denen jeweils verschiedene Gärstufen ablaufen. In der Regel weist eine Anlage zwei Fermenter und ein Gärrestelager auf. In dem ersten Fermenter läuft der Gärprozess in einer ersten Gärstufe ab, an deren Ende die Gärreste in einen zweiten Fermenter ausgetragen werden, in dem in einer zweiten Gärstufe ein Nachgärprozess stattfindet. Anschließend werden die Gärreste aus dem zweiten Fermenter ausgetragen und in das Gärrestelager überführt.

Jeder Fermenter weist mindestens eine Rohrleitung auf, um dem Fermenter ein fließfähiges Medium zuzuführen. Bei dem fließfähigen Medium kann es sich um Gärreste aus einem anderen Fermenter oder um ein fließfähiges Substrat, wie z. B. Gülle handeln. Ferner weist jeder Fermenter ein Abfuhrsystem für die Abfuhr der Gärreste aus dem Fermenter und mindestens einen Auslass für das bei dem Gärprozess entstehende Biogas auf.

Bei dem mikrobiellen Abbau des Substrats bildet sich Biogas als Stoffwechselprodukt von anaeroben Bakterien, insbesondere Methanbakterien, unter Ausschluss von Sauerstoff.

Bei der Erzeugung von Biogas kann der Gärprozess aus verschiedenen Gründen gestört werden und in Folge unvollständig ablaufen, so dass die Gasproduktion sinkt oder sich die Gaszusammensetzung verschlechtert. Dies kann bei einer Monovergärung von nährstoffarmen Substraten wie Mais auftreten und durch den Eintrag von Spurenelementen wie Kobalt, Nickel, Selen, Zink oder Molybdän oder durch den Eintrag von Makro-Nährstoffen wie Stickstoff, Phosphor oder Kalzium verhindert werden. Eine Prozesshemmung kann auch durch zu hohe Mengen von Substraten im Allgemeinen oder stickstoff- und schwefelhaltigen Substraten im Speziellen auftreten und durch Zusatzstoffe wie pH-regulierende Basen oder Säuren, durch Enzyme zur Aufspaltung von komplexen Substanzen oder durch andere Fermentationshilfsstoffe verhindert werden.

Einige der Zusatzstoffe können das Betriebspersonal oder die Umwelt gefährden und werden als Gefahrstoffe eingestuft. Es kann sich um krebserzeugende, keimzellmutagene oder reproduktionstoxische Gefahrstoffe handeln.

Das Lagern und der Einsatz von Gefahrstoffen in Produktionsanlagen, beispielsweise der Einsatz von Spurenelementen als Prozesshilfsmittel in Biogasanlagen, ist durch mehrere technische Regeln, insbesondere die Technischen Regeln für Gefahrstoffe (TRGS), reglementiert, um den Schutz des Personals und der Umwelt sicherzustellen. Im Fall von Gefahrstoffen beziehen sich die TRGS auf die Gefahrstoff-Verordnung (GefStoffV).

Beim Einsatz von Zusatzstoffen in Form von Spurenelementen, insbesondere Nickel-, Kobalt- und Selen-Salzen, bedeutet dies, dass
a) die Spurenelemente in staubdichten, jedoch wasserlöslichen Verpackungen, wie beispielsweise Beuteln oder Säcken in den Prozess eingetragen werden müssen, so dass die Spurenelemente erst im Fermenter bzw. Gärbehälter freigesetzt werden und ein Hautkontakt oder die Einatmung vermieden wird oder
b) die Spurenelemente durch Füllstoffe so stark verdünnt werden müssen, dass die Konzentrationen unter die Grenzwerte der GefStoffV fallen oder
c) die gefährlichen Spurenelemente durch die Bildung von Komplexen in ihrer Reaktivität gehemmt werden und somit nicht mehr als Gefahrstoff gelten.

Der Einsatz von Spurenelementen bei der Biogaserzeugung gemäß den Varianten b) und c) verursacht deutlich höhere Kosten als der Einsatz gemäß der Variante a). Bei der Variante b) verursachen der Füllstoff und das höhere Transportgewicht relativ hohe Kosten. Bei der Variante c) müssen Zusatzkosten für teure Komplexbildner aufgebracht werden. Die Kosten des Einsatzes der Spurenelemente bei der Biogaserzeugung können bei den Varianten b) und c) doppelt so hoch sein als die Kosten des Einsatzes gemäß Variante a).

Bei der Variante a) werden in der Praxis die Spurenelemente in einer staubdichten, wasserlöslichen Verpackung, die insbesondere als Beutel ausgebildet ist, in eine Rohrleitung eingebracht und mittels des fließfähigen Mediums, das sich in der Rohrleitung befindet, in den Fermenter eingetragen.

Es besteht die Gefahr, dass eine Verpackung in der Rohrleitung aufreißen kann und sich die Zusatzstoffe in Toträumen der Rohrleitung ansammeln können. Wenn kurz nach dem Eintragen von Zusatzstoffen, die als Gefahrstoffe eingestuft sind, eine Revision der Rohrleitung erforderlich wird, kann nicht ausgeschlossen werden, dass das Betriebspersonal in unerwünschter Weise in Kontakt mit den Gefahrstoffen kommen kann.

Neben den Risiken für das Betriebspersonal und die Umwelt aufgrund des Eintragens der Zusatzstoffe in den Prozess der Biogaserzeugung ist ein hoher betrieblicher Aufwand erforderlich, weil gemäß § 14 Abs. 3 Gefahrstoffverordnung bzw. TRGS 410 bei Tätigkeiten mit krebserzeugenden, keimzellmutagenen oder reproduktionstoxischen Gefahrstoffen der Kategorie 1A oder 1B u.a. ein Expositionsverzeichnis zu führen ist, was mit einem hohen betrieblichen Aufwand verbunden ist.

Vor dem oben beschriebenen Hintergrund liegt der Erfindung die Aufgabe zugrunde, eine Biogasanlage der eingangs genannten Art so zu verbessern sowie eine Einrichtung und ein Verfahren zum Eintragen von Zusatzstoffen in einen Fermenter zu schaffen, dass Zusatzstoffe sicher und unter Vermeidung von betrieblichem Aufwand aufgrund von Sicherheitsbestimmungen in den Fermenter eingetragen werden können, insbesondere derart, dass das Betriebspersonal und die Umwelt zu keinem Zeitpunkt gefährdet werden.

Die gestellte Aufgabe wird bei einer Biogasanlage der eingangs genannten Art durch die Merkmale des Patentanspruchs 1 gelöst.

Die erfindungsgemäße Biogasanlage ist dadurch gekennzeichnet, dass an die Rohrleitung stromab eines ersten Verschlussmittels ein Behälter für Zusatzstoffe angeschlossen ist, dass der Behälter mit einer Fluidleitung in Verbindung steht, welche ein Fluid führt, so dass die Zusatzstoffe mittels des Fluides in den Fermenter eintragbar sind.

Die Zusatzstoffe werden in einer staubdichten, wasserlöslichen Verpackung, insbesondere in Form von mindestens einem Beutel, durch die Rohrleitung in den Fermenter eingetragen.

Das erste Verschlussmittel in der Rohrleitung dient dazu, die Zufuhr des pumpfähigen Mediums zu dem Fermenter zu unterbrechen, um die Zusatzstoffe, die sich in dem Behälter befinden, mittels des Fluides durch die Rohrleitung stromab des ersten Verschlussmittels in den Fermenter einzutragen.

Spurenelemente, die wegen ihrer Toxizität und Mutagenität als Gefahrstoffe gelten, werden mittels des Fluides, in der Regel Wasser, während des Gärprozesses sicher in den Fermenter eingetragen, ohne dass das Betriebspersonal oder die Umwelt gefährdet werden.

Vorzugsweise weist der Behälter eine Einfüllöffnung und eine Auslassöffnung auf, wobei die Auslassöffnung mit einem zweiten Verschlussmittel versehen ist, um den Behälter von der ersten Rohrleitung zu trennen. Die Einfüllöffnung ist druckdicht verschließbar. Die Einfüllöffnung kann mittels eines beliebig ausgeführten Verschlussorgans druckdicht verschlossen werden. Vorzugsweise wird die Einfüllöffnung mittels eines druckdicht ausgeführten Deckels verschlossen.

Vorzugsweise ist der Behälter wenigstens teilweise als abgestumpfter Kegel oder als Zylinder ausgebildet.

Bei einer bevorzugten Weiterbildung ist die Fluidleitung an den Behälter benachbart zu dessen Einfüllöffnung angeschlossen, wobei sich in der Fluidleitung ein drittes Verschlussmittel befindet, um die Fluidleitung von dem Behälter zu trennen.

Der Behälter wird vorzugsweise direkt an die Rohrleitung angeschlossen, beispielsweise mittels eines kurzen Abzweiges, der von der Rohrleitung abgeht. Der Behälter wird vorzugsweise oben auf der Rohrleitung angeschlossen. Vorzugsweise verläuft die Achse des Behälter im Wesentlichen senkrecht zur Achse der Rohrleitung. Die Achse des Behälters bzw. der Behälter kann alternativ im unteren Bereich einen Krümmungsabschnitt aufweisen.

Alternativ kann der Behälter an die Rohrleitung mittels einer beliebig langen Anschlussleitung angeschlossen werden. Folglich kann sich zwischen dem Behälter und der Rohrleitung eine beliebig lange Anschlussleitung befinden und der Behälter räumlich getrennt von der Rohrleitung angeordnet werden.

In Weiterbildung der Erfindung ist die Biogasanlage nach der Erfindung dadurch gekennzeichnet, dass an den Behälter benachbart zu dessen Auslasslöffnung eine Fluidableitung zur Restentleerung des Fluids angeschlossen ist und sich in der Ableitung ein viertes Verschlussmittel befindet, um die Fluidableitung von dem Behälter zu trennen. Die Fluidableitung kann an einen Rohrstutzen angeschlossen sein. Das dritte Verschlussmittel ist vorzugsweise als Kugelhahn ausgebildet. In der Regel handelt es sich bei dem Fluid um Wasser, so dass die Fluidableitung zu einem Schmutzwasserrohr führt.

Nach einem weiteren Merkmal der Erfindung sind das erste, zweite, dritte und vierte Verschlussmittel regelbar.

Vorzugsweise ist die Rohrleitung an den Fermenter in Bodennähe angeschlossen. Die Rohrleitung kann aber auch auf andere Art und Weise an den Fermenter angeschlossen werden.

Die Erfindung schafft ferner eine Einrichtung zum Eintragen von Zusatzstoffen in einen Fermenter einer Biogasanlage, wobei an den Fermenter mindestens eine Rohrleitung zum Zuführen eines pumpfähigen Mediums in den Fermenter angeschlossen ist, dadurch gekennzeichnet, dass an die Rohrleitung stromab eines ersten Verschlussmittels ein Behälter für Zusatzstoffe angeschlossen ist und dass der Behälter mit einer Fluidleitung in Verbindung steht, welche ein Fluid führt, so dass die Zusatzstoffe mittels des Fluides in den Fermenter eintragbar sind. Die Einrichtung kann bei bestehenden Biogasanlagen nachgerüstet werden.

Die Erfindung beinhaltet ferner ein Verfahren zum Eintragen von Zusatzstoffen in einen Fermenter einer Biogasanlage, wobei an den Fermenter mindestens eine Rohrleitung zum Zuführen eines pumpfähigen Mediums in den Fermenter angeschlossen ist, dadurch gekennzeichnet, dass die Zufuhr des Substrats in den Fermenter mittels eines ersten Verschlussmittels unterbrochen wird, so dass die Zusatzstoffe, die sich in einem Behälter befinden, der an die Rohrleitung stromab des ersten Verschlussmittels angeschlossen ist, mittels eines Fluides aus einer Fluidleitung, die mit dem Behälter in Verbindung steht, in den Fermenter eingetragen werden.

Vorzugsweise werden die Zusatzstoffe in einer staubdichten, wasserlöslichen Verpackung, insbesondere in Form von mindestens einem Beutel, durch die Rohrleitung in den Fermenter eingetragen.

Nach einer bevorzugten Weiterentwicklung werden die Zusatzstoffe mittels eines vordefinierten Volumenstroms des Fluides in den Fermenter eingetragen. Der vordefinierte Volumenstrom entspricht vorzugsweise dem Volumen bzw. dem Totraum des Behälters plus des Volumens bzw. des Totraums der Rohrleitung ausgehend von der Anschlussstelle des Behälters an die Rohrleitung bis zum Fermenter.

Ein bevorzugtes Verfahren ist gekennzeichnet durch folgende aufeinander folgende Schritte:
a) Schließen eines ersten Verschlussmittels, welches sich in der Rohrleitung stromauf des Anschlusses des Behälters an die Rohrleitung befindet;
b) Befüllen des Behälters, der eine Einfüllöffnung und eine Auslassöffnung aufweist, wobei die Auslassöffnung mit einem zweiten Verschlussorgan verschlossen ist, mit Zusatzstoffen,
c) druckdichtes Verschließen der Einfüllöffnung des Behälters;
d) Öffnen eines dritten Verschlussmittels, welches sich in der Fluidleitung befindet, die an den Behälter angeschlossenen ist und Aufbauen eines Druckes, der größer ist als der hydrostatische Druck in der Rohrleitung, im Behälter;
e) Öffnen des zweiten Verschlussmittels und Eintragen der Zusatzstoffe mittels des Fluides durch die Rohrleitung in den Fermenter;
f) Schließen des zweiten und des dritten Verschlussmittels, Öffnen der Einfüllöffnung und Öffnen eines vierten Verschlussmittels in einer Fluidableitung, welche an den Behälter benachbart zu dessen Auslasslöffnung angeschlossen ist, zum Ablassen des Fluides aus dem Behälter zur Restentleerung.

Die Erfindung wird im Folgenden anhand eines bevorzugten Ausführungsbeispiels einer erfindungsgemäßen Biogasanlage sowie von bevorzugten Ausführungsformen einer Einrichtung zum Eintragen von Zusatzstoffen in einen Fermenter im Zusammenhang mit den Zeichnungen näher erläutert.

Die Zeichnungen zeigen in:
Fig. 1 eine schematische Darstellung einer ersten Ausführungsform einer Biogasanlage nach der Erfindung im Querschnitt;
Fig. 2 eine schematische Darstellung einer ersten Ausführungsform einer Einrichtung zum Eintragen von Zusatzstoffen in einen Fermenter aus Fig. 1 im Querschnitt;
Fig. 3 eine schematische Darstellung der Einrichtung aus Fig. 2 als Draufsicht;
Fig. 4 eine schematische Darstellung einer zweiten Ausführungsform einer Einrichtung zum Eintragen von Zusatzstoffen in einen Fermenter im Querschnitt;
Fig. 5 eine schematische Darstellung der zweiten Ausführungsform der Einrichtung aus Fig. 4 als Draufsicht.

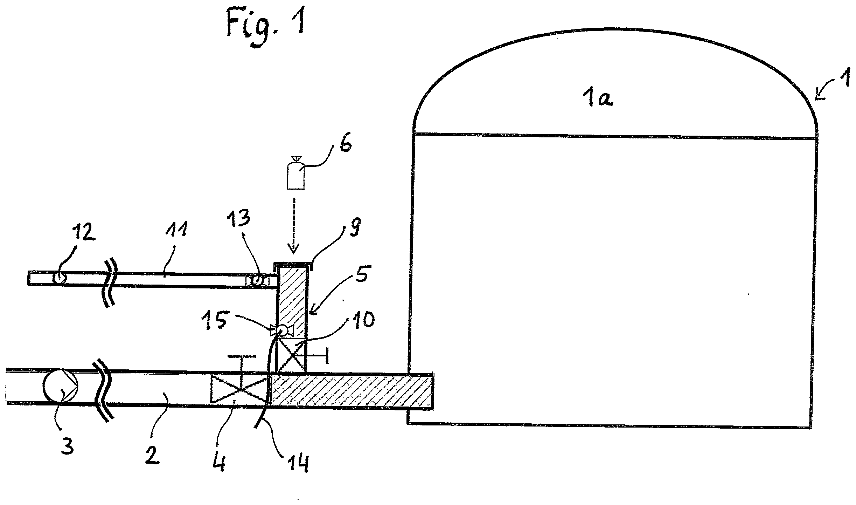

Fig. 1 zeigt schematisch eine Biogasanlage zum Erzeugen von Biogas aus einem Substrat. Die Anlage weist drei Fermenter bzw. Gärbehälter auf, von denen nur ein Fermenter 1 dargestellt ist. In diesem Fermenter erfolgt ein mikrobieller Abbau des Substrats während einer ersten Gärstufe. Wenn diese beendet ist, werden die Gärreste aus dem Fermenter 1 ausgetragen und in den zweiten, nicht dargestellten Fermenter zum Nachgären in eine zweite Gärstufe überführt. Nach Beendigung der zweiten Gärstufe werden die Gärreste aus dem zweiten Fermenter bzw. Nachgärer ausgetragen und in das Gärrestlager überführt. Die Fermenter und das Gärrestlager sind teilweise mit Substrat bzw. Biomasse bzw. mit Gärresten gefüllt.

Jeder Fermenter weist mindestens ein nicht dargestelltes Abfuhrsystem für die Abfuhr der Gärreste aus dem Fermenter und mindestens einen nicht dargestellten Auslass für das entstehende Biogas auf. Der erste Fermenter 1, der zweite Fermenter zum Nachgären und das Gärrestelager sind jeweils mit einem Gasspeicher 1a versehen, in dem sich das erzeugte Biogas sammelt und aus dem das Biogas abgeführt wird. Der Fermenter 1 und der Gasspeicher 1a bilden jeweils eine Baueinheit.

Jeder Fermenter 1 weist ferner mindestens eine Rohrleitung 2 auf, um dem Fermenter 1 ein fließfähiges Medium zuzuführen. Bei dem fließfähigen oder pumpfähigen Medium kann es sich um fließfähige Gärreste aus einem anderen Fermenter oder um ein fließfähiges Substrat, wie z. B. Gülle handeln. Das Medium wird mittels einer Pumpe 3, vorzugsweise einer Drehkolbenpumpe durch die Rohrleitung 2 in den Fermenter 1 gepumpt. Im Ausführungsbeispiel handelt es sich bei dem Medium um ein flüssiges Substrat, welches dem Fermenter 1 zum Vergären zugeführt wird. Die Rohrleitung 2 ist an den Fermenter 1 in Bodennähe angeschlossen und wird durch die Wand des Fermenters 1 ins Innere geführt. Die Rohrleitung 2 könnte aber auch auf andere Art und Weise an den Fermenter 1 angeschlossen werden.

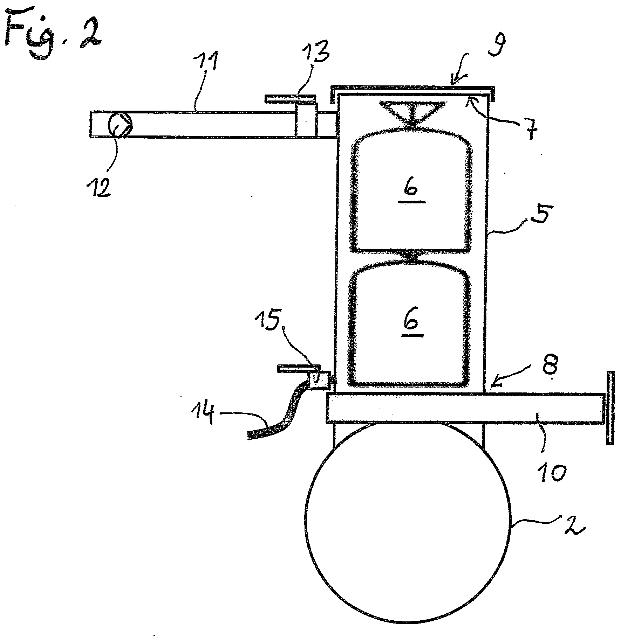

In der Rohrleitung 2 ist ein erstes Verschlussmittel 4 in Form eines Schiebers angeordnet. Stromab des ersten Verschlussmittels 4 ist ein Behälter 5 zum Beladen mit Zusatzstoffen 6 (Fig. 2) an die Rohrleitung 2 angeschlossen. Die Zusatzstoffe 6 befinden sich in mindestens einem staubdichten, aber wasserlöslichen Beutel, so dass das Betriebspersonal beim Beladen des Behälters 5 nicht mit den Zusatzstoffen 6 in Kontakt kommen kann.

Der Behälter 5 steht mit einer Fluidleitung 11 in Verbindung, welche ein Fluid führt. Im Ausführungsbeispiel handelt es sich bei dem Fluid um Wasser. Die Fluidleitung 11 ist drucklos, so dass eine zweite Pumpe 12, beispielsweise eine Kreiselpumpe, an die Fluidleitung 11 angeschlossen ist. Die Fluidleitung 11 ist an den Behälter 5 benachbart zu dessen Einfüllöffnung 7 angeschlossen. In der Fluidleitung 11 befindet sich ein drittes Verschlussmittel 13, um die Fluidleitung 11 von dem Behälter 5 trennen zu können.

Der Behälter 5 ist rohrförmig bzw. zylinderförmig und weist eine Einfüllöffnung 7 und eine Auslassöffnung 8 auf. Die Einfüllöffnung 7 ist druckdicht verschließbar. Bei dem Ausführungsbeispiel ist die Einfüllöffnung 7 mit einem Deckel 9 versehen.

Der Deckel 9 kann vorzugsweise mittels eines Scharniers, einer Dichtlippe und einer Arretierung druckdicht geschlossen werden. Die Druckfestigkeit bemisst sich an dem maximalen Druck, der durch die an den Behälter 5 angeschlossene Fluidleitung 11 aufgebaut wird und an dem maximalen Druck in der Rohrleitung 2.

Die Auslassöffnung 8 ist mit einem zweiten Verschlussmittel 10 in Form eines Kugelhahns oder eines Schiebers versehen, um den Behälter 5 von der ersten Rohrleitung 2 trennen zu können.

Der Durchmesser des Behälters 5 ist vorzugsweise gleich groß oder kleiner als der Durchmesser der Rohrleitung 2. Der Innendurchmesser des Behälters 5 ist gleich groß oder größer als der Durchmesser der Beutel oder Säcke mit den Zusatzstoffen 6, die in den Fermenter 1 eingetragen werden sollen.

An den Behälter 5 ist benachbart zu dessen Auslasslöffnung 8, vorzugsweise oberhalb des zweiten Verschlussmittels 10 und vorzugsweise an der tiefsten Stelle des Behälters 5 eine Fluidableitung 14, zum Ableiten des Fluides bzw. des Wassers aus dem Behälter, nachdem die Zusatzstoffe 6 in den Fermenter 1 eingetragen worden sind, angeschlossen. In der Fluidableitung 14 befindet sich ein viertes Verschlussmittel 15 in Form eines Kugelhahns, um die Fluidableitung 14 von dem Behälter 5 trennen zu können. Die Fluidableitung 14 führt im Ausführungsbeispiel zu einem nicht dargestellten Schmutzwasserrohr. Alternativ kann die Fluidableitung 14 zu einem Speicher für das abgeführte Fluid führen.

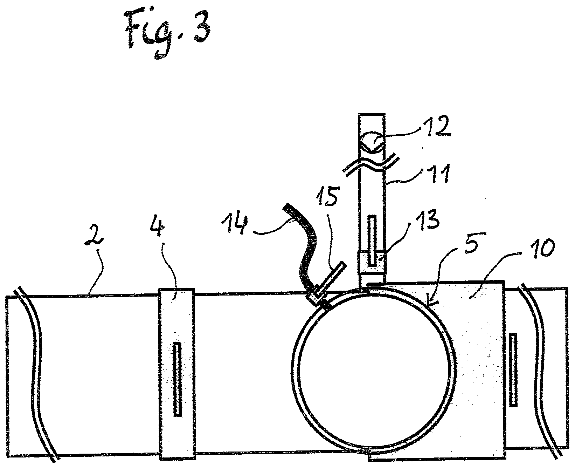

Bei der Einrichtung in Fig. 2 und 3 ist dargestellt, dass die Achse des Behälter 5 im Wesentlichen senkrecht zur Achse der Rohrleitung 2 verläuft. Der Behälter ist direkt mittig oben auf der Rohrleitung angeordnet. Der Durchmesser des Behälters 5 ist etwas kleiner als der Durchmesser der Rohrleitung 2.

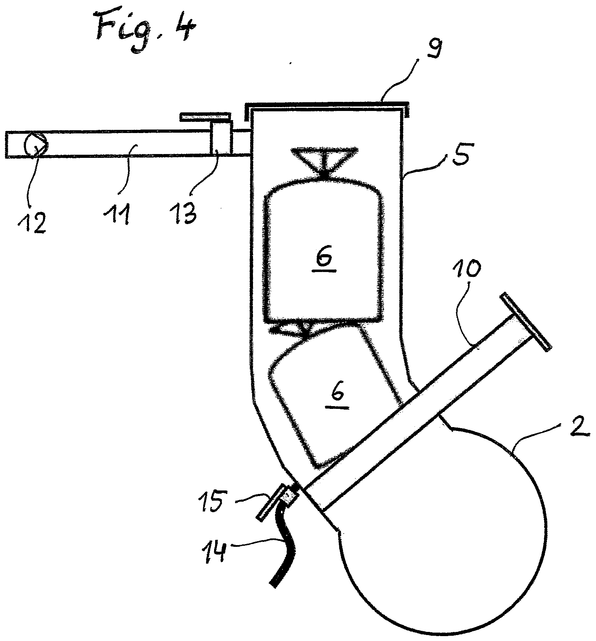

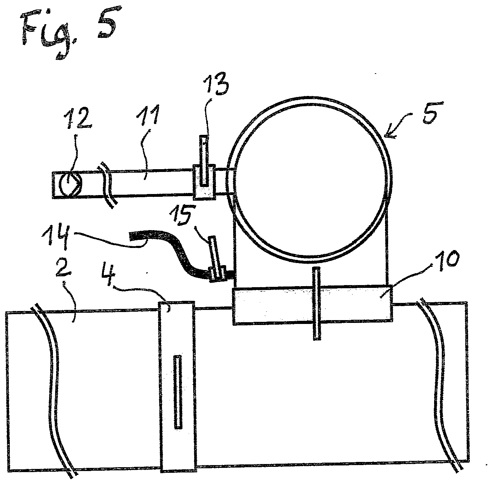

Bei dem in Fig. 4 und 5 dargestellten Ausführungsform der Einrichtung nach der Erfindung weist der Behälter 5 im Bereich des Anschlusses an die Rohrleitung 2 einen Krümmungsabschnitt auf.

Der Eintrag der Zusatzstoffe 6 in den Fermenter 1 wird wie folgt durchgeführt:
Die erste Pumpe 3 in der Rohrleitung 2 wird abgestellt und das erste Verschlussmittel 4, das sich stromab der Pumpe 3 in Rohrleitung 2 befindet, wird geschlossen. Danach wird das zweite Verschlussmittel 10, welches den Behälter 5 von der Rohrleitung 2 trennt, geschlossen bzw. sichergestellt, dass das zweite Verschlussmittel 10 geschlossen ist. Das dritte Verschlussmittel 13 in der Fluidleitung 11 und das vierte Verschlussmittel 15 in der Fluidableitung 14 sind ebenfalls geschlossen. Dann wird der Deckel 9 des Behälters 5 geöffnet und die Zusatzstoffe 6 in staubdichten, wasserlöslichen Beuteln bzw. Packungen in den leeren Behälter 5 gefüllt. Anschließend wird mittels des Deckels 9 der Behälter 5 druckdicht verschlossen. Durch Öffnen des dritten Verschlussmittels 13 in der Fluidleitung 11, ggf. durch Einschalten der zweiten Pumpe 12, wird ein Fluid, hier Wasser, in den Behälter 5 geleitet und ein Druck aufgebaut, der größer ist als der hydrostatische Druck in der Rohrleitung 2, welche an den Fermenter 1 bzw. Gärbehälter angeschlossen ist.

Anschließend wird das zweite Verschlussmittel 10 zwischen dem Behälter 5 und der Rohrleitung 2 geöffnet und die Beutel bzw. Packungen mit den Zusatzstoffen 6 durch die Rohrleitung 2 in den Fermenter 1 bzw. Gärbehälter gespült. Hierbei ist es hilfreich, wenn der Innendurchmesser des Behälters 5 so gewählt wird, dass der oder die einzutragenden Beutel oder Packungen als Pfropfen eingetragen werden.

Ohne Pfropfenstrom-Eintrag sinken der oder die Beutel durch Wirkung der Schwerkraft und des angelegten Wasserdrucks in die Rohrleitung 2.

Es wird vorzugsweise mindestens die Menge Wasser eingeleitet, die dem Totraum des Behälters 5 (schraffiert dargestellt) entspricht, d. h. dem Volumen zwischen dem Deckel 9 des Behälters 5 und dem zweiten Verschlussmittel 10 plus dem Totraum der Rohrleitung 2 (schraffiert dargestellt), d. h. dem Volumen der Rohrleitung 2 ausgehend vom zweiten Verschlussmittel 10 bis zum Ende der Rohrleitung 2 im Fermenter 1. Auf diese Weise wird sichergestellt, dass ein Pfropfen von Beuteln bis an das Ziel gespült wird und etwaige Kontaminationen der Bestandsleitung unter den Grenzwert der Gefahrstoffe verdünnt wird.

Anschließend wird das zweite Verschlussmittel 10 zwischen dem Behälter 5 und der Rohrleitung 2 und das dritte Verschlussmittel 13 in der Fluidleitung 11 geschlossen. Nun kann weder kontaminiertes Wasser oder Substrat bzw. Gärmedium in den Behälter 5 zurückströmen, noch wird neues Wasser aus der Fluidleitung 11 in den Behälter 5 eingetragen.

Im letzten Schritt wird das vierte Verschlussmittel 15 in der Fluidableitung 14 geöffnet, so dass das Wasser durch die Fluidableitung 14 aus dem Behälter 5 ablaufen kann und ggf. für einen neuerlichen Zyklus zur Verfügung steht.

Bei einem geringen inneren Durchmessern der Fluidableitung 14 kann der Deckel 9 des Behälters 5 geöffnet werden und so der Aufbau eines Unterdrucks im Behälter 5 verhindert werden. Wenn Behälter 5 leer ist, wird das vierte Verschlussmittel 15 in der Fluidableitung 14 wieder geschlossen. Schließlich wird das erste Verschlussmittel 4 in der Rohrleitung 2 geöffnet, so dass die Rohrleitung 2 wieder für den ursprünglichen Zweck verwendet werden kann,

Im Rahmen der Erfindung sind ohne weiteres Abwandlungen möglich: So kann der Behälter 5 wenigstens teilweise als abgestumpfter Kegel ausgebildet sein. Zwischen der Austrittsöffnung 8 des Behälters 5 und der Rohrleitung 2 kann sich eine beliebig lange Anschlussleitung befinden.

Merkmale oder Schritte, die mit Verweis auf eines der obigen Ausführungsbeispiele beschrieben worden sind, können auch in Kombination mit anderen Merkmalen oder Schritten anderer beschriebener Ausführungsbeispiele verwendet werden. Schließlich wird darauf hingewiesen, dass "aufweisend" keine anderen Elemente oder Schritte ausschließt, dass "eine" oder "ein" keine Vielzahl ausschließt und Bezugszeichen in den Ansprüchen nicht als Einschränkung anzusehen sind.

**Bezugszeichenliste**

| | |
|---|---|
| 1 | Fermenter |
| 1a | Gasspeicher |
| 2 | Rohrleitung |
| 3 | erste Pumpe |
| 4 | erstes Verschlussmittel |
| 5 | Behälter |
| 6 | Zusatzstoffe |
| 7 | Einfüllöffnung |
| 8 | Auslassöffnung |
| 9 | Deckel |
| 10 | zweites Verschlussmittel |
| 11 | Fluidleitung |
| 12 | zweite Pumpe |
| 13 | drittes Verschlussmittel |
| 14 | Fluidableitung |
| 15 | viertes Verschlussmittel |

## Patentansprüche

1. Biogasanlage, mit mindestens einem Fermenter (1) für den Abbau eines Substrats zum Erzeugen von Biogas, wobei an den Fermenter (1) mindestens eine Rohrleitung (2) zum Zuführen eines pumpfähigen Mediums in den Fermenter (1) angeschlossen ist,
**dadurch gekennzeichnet,**
**dass** an die Rohrleitung (2) stromab eines ersten Verschlussmittels (4) ein Behälter (5) für Zusatzstoffe (6) angeschlossen ist, dass der Behälter (5) mit einer Fluidleitung (11) in Verbindung steht, welche ein Fluid führt, so dass die Zusatzstoffe (6) mittels des Fluides in den Fermenter (1) eintragbar sind.

2. Biogasanlage nach Anspruch 1,
**dadurch gekennzeichnet, dass** der Behälter (5) eine Einfüllöffnung (7) und eine Auslassöffnung (8) aufweist und dass die Auslassöffnung (8) mit einem zweiten Verschlussmittel (10) versehen ist, um den Behälter (5) von der ersten Rohrleitung (2) zu trennen.

3. Biogasanlage nach Anspruch 2,
**dadurch gekennzeichnet, dass** die Einfüllöffnung (7) druckdicht verschließbar ist, insbesondere mittels eines druckdichten Deckels (9).

4. Biogasanlage nach wenigstens einem der vorhergehenden Ansprüche,
**dadurch gekennzeichnet, dass** der Behälter (5) wenigstens teilweise als abgestumpfter Kegel oder als Zylinder ausgebildet ist.

5. Biogasanlage nach wenigstens einem der vorangegangenen Ansprüche,
**dadurch gekennzeichnet, dass** die Fluidleitung (11) an den Behälter (5) benachbart zu dessen Einfüllöffnung (7) angeschlossen ist und dass sich in der Fluidleitung (11) ein drittes Verschlussmittel (13) befindet, um die Fluidleitung (11) von dem Behälter (5) zu trennen.

6. Biogasanlage nach wenigstens einem der vorhergehenden Ansprüche,
**dadurch gekennzeichnet, dass** die Achse des Behälters (5) im Wesentlichen senkrecht zur Achse der Rohrleitung (2) verläuft oder das die Achse im unteren Bereich des Behälters einen Krümmungsabschnitt aufweist.

7. Biogasanlage nach wenigstens einem der vorangegangenen Ansprüche,
**dadurch gekennzeichnet, dass** der Behälter (5) mittels einer Anschlussleitung an die Rohrleitung (2) angeschlossen ist.

8. Biogasanlage nach wenigstens einem der vorhergehenden Ansprüche,
**dadurch gekennzeichnet, dass** an den Behälter (5) benachbart zu dessen Auslasslöffnung (8) eine Fluidableitung (14) für die Ableitung des Fluids angeschlossen ist und sich in der Fluidableitung (14) ein viertes Verschlussmittel (15) befindet, um die Fluidableitung (14) von dem Behälter (5) zu trennen.

9. Biogasanlage nach wenigstens einem der vorhergehenden Ansprüche,
**dadurch gekennzeichnet, dass** das erste, zweite, dritte und vierte Verschlussmittel (4, 10, 13, 15) regelbar sind.

10. Biogasanlage nach wenigstens einem der vorhergehenden Ansprüche,
**dadurch gekennzeichnet, dass** die Rohrleitung (2) an den Fermenter (1) in Bodennähe angeschlossen ist.

11. Einrichtung zum Eintragen von Zusatzstoffen in einen Fermenter einer Biogasanlage, wobei an den Fermenter (1) mindestens eine Rohrleitung (2) zum Zuführen eines pumpfähigen Mediums in den Fermenter (1) angeschlossen ist, **dadurch gekennzeichnet, dass** an die Rohrleitung (2) stromab eines ersten Verschlussmittels (4) ein Behälter (5) für Zusatzstoffe (6) angeschlossen ist und dass der Behälter (5) mit einer Fluidleitung (11) in Verbindung steht, welche ein Fluid führt, so dass die Zusatzstoffe (6) mittels des Fluides in den Fermenter (1) eintragbar sind.

12. Verfahren zum Eintragen von Zusatzstoffen in einen Fermenter einer Biogasanlage, wobei an den Fermenter (1) mindestens eine Rohrleitung (2) zum Zuführen eines pumpfähigen Mediums in den Fermenter (1) angeschlossen ist,
**dadurch gekennzeichnet,**
**dass** die Zufuhr des Substrats in den Fermenter (1) mittels eines ersten Verschlussmittels (4) unterbrochen wird, dass die Zusatzstoffe (6), die sich in einem Behälter (5) befinden, der an die Rohrleitung (2) stromab des ersten Verschlussmittels (4) angeschlossen ist, mittels eines Fluides aus einer Fluidleitung (11), welche mit dem Behälter (5) in Verbindung steht, in den Fermenter (1) eingetragen werden.

13. Verfahren nach Anspruch 12,
**dadurch gekennzeichnet, dass** die Zusatzstoffe (6) in einer staubdichten, wasserlöslichen Verpackung, insbesondere in Form von mindestens einem Beutel, durch die Rohrleitung (2) in den Fermenter (1) eingetragen werden.

14. Verfahren nach Anspruch 12 oder 13,
**dadurch gekennzeichnet, dass** die Zusatzstoffe (6) mittels eines vordefinierten Volumenstroms des Fluides in den Fermenter (1) eingetragen werden.

15. Verfahren nach wenigstens einem der Ansprüche 12 bis 14,
**gekennzeichnet durch** folgende aufeinander folgenden Schritte:
a) Schließen des ersten Verschlussmittels (4), welches sich in der Rohrleitung (2) stromauf des Anschlusses des Behälters (5) an die Rohrleitung (2) befindet;
b) Befüllen des Behälters (5), der eine Einfüllöffnung (7) und eine Auslassöffnung (8) aufweist, wobei die Auslassöffnung (8) mit einem zweiten Verschlussmittel (10) verschlossen ist, mit Zusatzstoffen (6);
c) druckdichtes Schließen der Einfüllöffnung (7) des Behälters (5);
d) Öffnen eines dritten Verschlussmittels (13), welches sich in der Fluidleitung (11) befindet, die an den Behälter (5) angeschlossenen ist und Aufbauen eines Druckes, der größer ist als der hydrostatische Druck in der Rohrleitung (2), im Behälter (5);
e) Öffnen des zweiten Verschlussmittels (10) und Eintragen der Zusatzstoffe (6) mittels des Fluides durch die Rohrleitung (2) in den Fermenter (1);
f) Schließen des zweiten und des dritten Verschlussmittels (10, 13), Öffnen der Einfüllöffnung (7) und Öffnen eines vierten Verschlussmittels in einer Fluidableitung (14), welche an den Behälter (5) benachbart zu dessen Auslasslöffnung (8) angeschlossen ist, zum Ablassen des Fluides aus dem Behälter (5) zur Restentleerung.

## Claims

1. Biogas plant, having at least one fermenter (1) for decomposing a substrate for producing biogas, with at least one pipeline (2) for feeding a pumpable medium to the fermenter (1) being connected to the fermenter (1),
**characterised in that**,
a container (5) for additives (6) is connected to the pipeline (2) downstream of a first closure means (4), **in that** the container (5) communicates with a fluid line (11) which carries a fluid, so that the additives (6) are introducible into the fermenter (1) by means of the fluid.

2. Biogas plant according to claim 1,
**characterised in that** the container (5) exhibits a filling opening (7) and an outlet opening (8) and that the outlet opening (8) is provided with a second closure means (10) to separate the container (5) from the first pipeline (2).

3. Biogas plant according to claim 2,
**characterised in that** the filling opening (7) can be closed in a pressure-tight manner, in particular by means of a pressure-tight lid (9).

4. Biogas plant according to at least one of the preceding claims,
**characterised in that** the container (5) is at least partially formed as a blunted cone or as a cylinder.

5. Biogas plant according to at least one of the preceding claims,
**characterised in that** the fluid line (11) is connected to the container (5) adjacent to the filling opening (7) thereof, and **in that** a third closure means (13) is located in the fluid line (11) to separate the fluid line (11) from the container (5).

6. Biogas plant according to at least one of the preceding claims,
**characterised in that** the axis of the container (5) is substantially perpendicular to the axis of the pipeline (2) or **in that** the axis exhibits a curved section in the lower area of the container.

7. Biogas plant according to at least one of the preceding claims,
**characterised in that** the container (5) is connected to the pipeline (2) by means of a connecting line.

8. Biogas plant according to at least one of the preceding claims,
**characterised in that** a fluid drain line (14) for draining the fluid is connected to the container (5) adjacent to the outlet opening (8) thereof, and a fourth closure means (15) is located in the fluid drain line (14) to separate the fluid drain line (14) from the container (5).

9. Biogas plant according to at least one of the preceding claims,
**characterised in that** the first, second, third and fourth closure means (4, 10, 13, 15) are controllable.

10. Biogas plant according to at least one of the preceding claims,
**characterised in that** the pipeline (2) is connected to the fermenter (1) near the ground.

11. Device for introducing additives into a fermenter of a biogas plant, with at least one pipeline (2) for feeding a pumpable medium into the fermenter (1) being connected to the fermenter (1), **characterised in that** the pipeline (2) is connected to the fermenter (1), **in that** a container (5) for additives (6) is connected to the pipeline (2) downstream of a first closure means (4), and **in that** the container (5) communicates with a fluid line (11) which carries a fluid, so that the additives (6) can be introduced into the fermenter (1) by means of the fluid.

12. Method for introducing additives into a fermenter of a biogas plant, with at least one pipeline (2) for feeding a pumpable medium into the fermenter (1) being connected to the fermenter (1),
**characterised in that**,
the feeding of the substrate into the fermenter (1) is interrupted by means of a first closure means (4), **in that** the additives (6), which are located in a container (5) connected to the pipeline (2) downstream of the first closure means (4), are introduced into the fermenter (1) by means of a fluid from a fluid line (11) which communicates with the container (5).

13. Method according to claim 12,
**characterised in that** the additives (6) are introduced into the fermenter (1) through the pipeline (2) in a dust-tight, water-soluble package, in particular in the form of at least one bag.

14. Method according to claim 12 or claim 13,
**characterised in that** the additives (6) are introduced into the fermenter (1) by means of a predefined volume flow of the fluid.

15. Method according to at least one of claims 12 to 14,
**characterised by** the following successive steps:
(a) closing the first closure means (4) which is located in the pipeline (2) upstream of the connection of the container (5) to the pipeline (2);
(b) filling the container (5), which exhibits a filling opening (7) and an outlet opening (8), the outlet opening (8) being closed by a second closure means (10), with additives (6);
(c) closing the filling opening (7) of the container (5) in a pressure-tight manner;
(d) opening a third closure means (13) located in the fluid line (11) connected to the container (5) and building up a pressure in the container (5) which is greater than the hydrostatic pressure in the pipeline (2);
(e) opening the second closure means (10) and introducing the additives (6) by means of the fluid through the pipeline (2) into the fermenter (1);
(f) closing the second and the third closure means (10, 13), opening the filling opening (7) and opening a fourth closure means in a fluid drain line (14) connected to the container (5) adjacent to its outlet opening (8) for draining the fluid from the container (5) for residue removal.

## Revendications

1. Installation de production de biogaz, comportant au moins un fermenteur (1) pour la décomposition d'un substrat destiné à la production de biogaz, dans laquelle au moins une canalisation (2) pour l'amenée d'un milieu pompable dans le fermenteur (1) est raccordée au fermenteur (1),
**caractérisée en ce**
**qu'**un récipient (5) pour des additifs (6) est raccordé à la canalisation (2) en aval d'un premier moyen de fermeture (4), que le récipient (5) est relié à une conduite de fluide (11) qui transporte un fluide, de telle sorte que les additifs (6) peuvent être introduits dans le fermenteur (1) au moyen du fluide.

2. Installation de production de biogaz selon la revendication 1,
**caractérisée en ce que** le récipient (5) présente une ouverture de remplissage (7) et une ouverture de sortie (8) et que l'ouverture de sortie (8) est pourvue d'un deuxième moyen de fermeture (10) pour séparer le récipient (5) de la première canalisation (2).

3. Installation de production de biogaz selon la revendication 2,
**caractérisée en ce que** l'ouverture de remplissage (7) peut être fermée de manière étanche à la pression, en particulier au moyen d'un couvercle étanche à la pression (9).

4. Installation de production de biogaz selon au moins l'une des revendications précédentes,
**caractérisée en ce que** le récipient (5) est réalisé au moins partiellement sous la forme d'un cône tronqué ou d'un cylindre.

5. Installation de production de biogaz selon au moins l'une des revendications précédentes,
**caractérisée en ce que** la conduite de fluide (11) est raccordée au récipient (5) à proximité de son ouverture de remplissage (7) et qu'un troisième moyen de fermeture (13) est situé dans la conduite de fluide (11) pour séparer la conduite de fluide (11) du récipient (5).

6. Installation de production de biogaz selon au moins l'une des revendications précédentes,
**caractérisée en ce que** l'axe du récipient (5) est sensiblement perpendiculaire à l'axe de la canalisation (2) ou que l'axe présente une partie incurvée dans la zone inférieure du récipient.

7. Installation de production de biogaz selon au moins l'une des revendications précédentes,
**caractérisée en ce que** le récipient (5) est raccordé à la canalisation (2) au moyen d'une conduite de raccordement.

8. Installation de production de biogaz selon au moins l'une des revendications précédentes,
**caractérisée en ce qu'**une conduite d'évacuation de fluide (14) pour évacuer le fluide est raccordée au récipient (5) à proximité de son ouverture de sortie (8) et qu'un quatrième moyen de fermeture (15) est situé dans la conduite d'évacuation de fluide (14) pour séparer la conduite d'évacuation de fluide (14) du récipient (5).

9. Installation de production de biogaz selon au moins l'une des revendications précédentes,
**caractérisée en ce que** les premier, deuxième, troisième et quatrième moyens de fermeture (4, 10, 13, 15) sont réglables.

10. Installation de production de biogaz selon au moins l'une des revendications précédentes,
**caractérisée en ce que** la canalisation (2) est raccordée au fermenteur (1) à proximité du fond.

11. Dispositif pour introduire des additifs dans un fermenteur d'une installation de production de biogaz, dans lequel au moins une canalisation (2) pour l'amenée d'un milieu pompable dans le fermenteur (1) est raccordée au fermenteur (1), **caractérisé en ce qu'**un récipient (5) pour des additifs (6) est raccordé à la canalisation (2) en aval d'un premier moyen de fermeture (4) et que le récipient (5) est relié à une conduite de fluide (11) qui transporte un fluide, de telle sorte que les additifs (6) peuvent être introduits dans le fermenteur (1) au moyen du fluide.

12. Procédé pour introduire des additifs dans un fermenteur d'une installation de production de biogaz, dans lequel au moins une canalisation (2) pour l'amenée d'un milieu pompable dans le fermenteur (1) est raccordée au fermenteur (1), **caractérisé en ce**
**que** l'amenée du substrat dans le fermenteur (1) est interrompue au moyen d'un premier moyen de fermeture (4), que les additifs (6) qui se trouvent dans un récipient (5), qui est raccordé à la canalisation (2) en aval du premier moyen de fermeture (4), sont introduits dans le fermenteur (1) au moyen d'un fluide provenant d'une conduite de fluide (11) qui est reliée au récipient (5).

13. Procédé selon la revendication 12,
**caractérisé en ce que** les additifs (6) sont introduits dans le fermenteur (1) par la canalisation (2) dans un emballage étanche à la poussière et soluble dans l'eau, en particulier sous la forme d'au moins un sachet.

14. Procédé selon la revendication 12 ou 13,
**caractérisé en ce que** les additifs (6) sont introduits dans le fermenteur (1) au moyen d'un débit volumique prédéfini du fluide.

15. Procédé selon au moins l'une des revendications 12 à 14,
**caractérisé par** les étapes successives suivantes :
a) fermeture du premier moyen de fermeture (4) qui est situé dans la canalisation (2) en amont du raccordement du récipient (5) à la canalisation (2) ;
b) remplissage avec des additifs (6) du récipient (5) qui présente une ouverture de remplissage (7) et une ouverture de sortie (8), l'ouverture de sortie (8) étant fermée par un deuxième moyen de fermeture (10) ;
c) fermeture étanche à la pression de l'ouverture de remplissage (7) du récipient (5) ;
d) ouverture d'un troisième moyen de fermeture (13) qui est situé dans la conduite de fluide (11) qui est raccordée au récipient (5) et établissement d'une pression supérieure à la pression hydrostatique dans la canalisation (2) du récipient (5) ;
e) ouverture du deuxième moyen de fermeture (10) et introduction des additifs (6) au moyen du fluide par la canalisation (2) dans le fermenteur (1) ;
f) fermeture des deuxième et troisième moyens de fermeture (10, 13), ouverture de l'ouverture de remplissage (7) et ouverture d'un quatrième moyen de fermeture dans une conduite d'évacuation de fluide (14) qui est raccordée au récipient (5) à proximité de son ouverture de sortie (8) pour évacuer le fluide du récipient (5) et permettre sa vidange complète.
